# EUROPEAN PATENT SPECIFICATION

(11) **EP 3 763 345 B1**
(45) Date of publication and mention of the grant of the patent: **03.04.2024**
(21) Application number: 19763152.6
(22) Date of filing: 19.02.2019
(51) Int. Cl.: A61F 13/533, A61F 13/47, A61F 13/472, A61F 13/532, A61F 13/536, D04H 3/007, D04H 3/14, D04H 3/16, A61F 13/475, A61F 13/537

(54) **ABSORBENT ARTICLE**
ABSORBIERENDER ARTIKEL
ARTICLE ABSORBANT

(30) Priority: 05.03.2018 JP 2018038352
(43) Date of publication of application: 13.01.2021
(73) Proprietor: DAIO PAPER CORPORATION, Shikokuchuo-shi Ehime 799-0492 (JP)
(72) Inventor: SUYAMA, Junnosuke, Sakura-shi, Tochigi 329-1411 (JP)
(74) Representative: Epping - Hermann - Fischer
(86) International application number: PCT/JP2019/006006
(87) International publication number: WO 2019/171934

(56) References cited:
- WO-A1-2012/118214
- WO-A1-2013/047715
- WO-A1-2014/073396
- WO-A1-2016/157490
- WO-A1-2017/187669
- JP-A- 2003 126 140
- JP-A- 2013 075 082
- JP-U- H 069 616
- JP-U- H03 101 932

## Description

### TECHNICAL FIELD

The present invention relates to an absorbent article mainly used for products such as sanitary napkins.

### BACKGROUND ART

There is a known absorbent article where an absorber formed of, for example, cotton pulp is disposed between an impermeable back-side sheet such as a polyethylene sheet or a polyethylene-sheet-laminated nonwoven fabric and a permeable upper-side sheet such as a nonwoven fabric or a permeable plastic sheet.

A slim absorbent article including a thin absorber has such advantages that wearing discomfort can be reduced and that the bumps of the absorbent article are less noticeable from the outside of clothes when the absorbent article is worn (i.e., the absorbent article has less influence on the outer appearance), and is therefore popular in the market.

However, because the absorber is thin, the absorption capacity per unit area is small; and because the fluid retaining capacity in the thickness direction is low, the body fluid is likely to diffuse in the plane direction. For this reason, the related-art absorbent article has a disadvantage that the body fluid tends to leak particularly from a lateral edge of the absorber (side leakage). This in turn causes the wearer to feel insecure about the leakage and causes inconvenience such as having to replace absorbent articles frequently.

There is a known technology for preventing such side leakage in which compressed grooves are formed along the longitudinal direction on sides of an area corresponding to a body fluid discharge part of the wearer by denting the absorbent article from the skin side of the upper-side sheet toward the non-skin side, and there are many documents related to this technology. For example, Patent Document 1 discloses an absorbent article where a reference mark, which is a continuous or discontinuous contour line surrounding a predetermined area of a fluid receiving part, is provided in a position visible from the front side.

Also, Patent Document 2 discloses an absorbent article including an absorber where multiple compressed parts are formed by compressing an upper-side sheet and the absorber in the thickness direction of the absorbent article. The compressed parts include dot-shaped central compressed parts that are intermittently formed and disposed in a central area including the center of an excretion opening contact area facing an excretion opening of a wearer, and longitudinal compressed parts that are disposed outside of the central area in the width direction of the absorbent article and extend in the longitudinal direction of the absorbent article. The longitudinal compressed parts are depressed from the upper-side sheet toward the back-side sheet, and the central compressed parts are depressed from the back-side sheet toward the upper-side sheet.

### [Related-Art Documents]

### [Patent Documents]

[Patent Document 1] Japanese Unexamined Patent Application Publication No. 2003-126140
[Patent Document 2] Japanese Unexamined Patent Application Publication No. 2013-034525

WO 2012/118214 A1 relates to an absorbent.
WO 2014/073396 A1 relates to absorbent articles such as sanitary products, incontinence products and disposable diapers.
WO 2017/187669 A1 relates to an absorbent article.
WO 2013/047715 A1 relates to absorbent articles such as sanitary napkins, panty liners, incontinence pads and the like for absorbing menstrual blood and vaginal discharge, and more specifically, a concave portion formed of a combination of a wavy pattern and a dot pattern on a liquid-permeable surface sheet It is related with the absorbent article which formed.

### DISCLOSURE OF INVENTION

### PROBLEMS TO BE SOLVED BY THE INVENTION

However, because the absorbent article described in Patent Document 1 does not use a thin absorber, the absorbent article is likely to cause the wearer to feel discomfort and tends to influence the outer appearance. Also, although the absorbent article described in Patent Document 2 includes multiple dot-shaped central compressed parts, the central compressed parts are provided to prevent the upper-side sheet from being damaged by heat and to draw in a body fluid discharged from the wearer by the upper-side sheet, and do not have much effect in reducing the thickness of the absorber. Thus, the absorbent article of Patent Document 2 also has disadvantages similar to those of the absorbent article of Patent Document 1.

Thus, Patent Documents 1 and 2 are not directed to an absorbent article using a thin absorber, and therefore do not teach problems of a thin absorber that has a small absorption capacity per unit area and where a body fluid tends to diffuse in the plane direction and side leakage tends to occur.

A main object of the present invention is to provide a slim absorbent article that uses a thin absorber and where the diffusion of a body fluid in the plane direction is suppressed.

### MEANS FOR SOLVING THE PROBLEMS

To solve the above problems, an aspect of the present invention provides an absorbent article according to claim 1.

According to the embodiment, the thickness of the absorber is reduced in the thickness direction by forming the core press on the absorber. In an absorber with a core press, the body fluid may diffuse along the core press where the density of fibers is increased by compression, and leakage (side leakage) from the lateral edges may occur depending on the use condition. However, in the present invention, right and left compressed grooves are formed along the longitudinal direction of the absorbent article on at least the sides of the area corresponding to the body fluid discharge part of the wearer to block the diffusion of the body fluid to the lateral edges of the absorber, and a high absorption part having a greater absorption capacity per unit area than other parts is formed at least between the right and left compressed grooves of the absorber. This configuration makes it possible to prevent the diffusion of a discharged body fluid in the plane direction and to absorb and retain the body fluid in the absorber.

Accordingly, this embodiment makes it possible to provide a slim absorbent article that uses a thin absorber and where the diffusion of a body fluid in the plane direction, particularly in the width direction, is suppressed.

According to an embodiment, the compressed grooves are formed to surround the area corresponding to the body fluid discharge part of the wearer.

According to the embodiment the compressed grooves are formed to surround the area corresponding to the body fluid discharge part of the wearer. This embodiment makes it possible to suppress the diffusion in the longitudinal direction in addition to the diffusion in the width direction, and thereby makes it possible to reliably prevent the leakage from the edges of the absorber.

According to an embodiment, multiple compressed grooves are formed at least on each side of the area corresponding to the body fluid discharge part of the wearer, and the absorption capacity per unit area of the absorber in an inner area inside of the compressed grooves is greater than the absorption capacity per unit area of the absorber in an outer area outside of the compressed grooves.

In the embodiment, when multiple compressed grooves are formed on each side, the absorption capacity per unit area is changed gradually in areas bordered by the compressed grooves. This configuration makes it possible to further suppress the diffusion of the body fluid and more reliably prevent the side leakage. The absorption capacity per unit area can be adjusted by changing the basis weight of the pulp and the superabsorbent polymer or by changing the types of superabsorbent polymers.

According to an embodiment, the core press includes multiple linear pressed parts that are formed between edges of the absorber and are continuous or omitted in the compressed grooves.

In the embodiment, the core press includes the linear pressed parts formed between the edges of the absorber. This makes it possible to reliably reduce the thickness of the absorber.

According to an embodiment, the core press includes multiple first linear pressed parts that are arranged at predetermined intervals in the longitudinal direction and inclined to one side in a width direction, and multiple second linear pressed parts that are arranged in the longitudinal direction at predetermined intervals, inclined to another side in the width direction, and intersect the first linear pressed parts.

In the embodiment, the core press is formed in a grid-like pattern including the first linear pressed parts and the second linear pressed parts inclined in different directions. With this configuration, the body fluid diffuses along the first linear pressed parts and the second linear pressed parts. This in turn makes it possible to suppress the diffusion in the width direction, and enables the absorber to smoothly deform in an oblique direction along the first linear pressed parts and the second linear pressed parts and to smoothly follow the movement of the body when the absorber is twisted.

According to an embodiment, the groove width of the compressed grooves is greater than the groove width of the linear pressed parts.

In the embodiment, the groove width of the compressed grooves is made greater than the groove width of the linear pressed parts so that the body fluid reaching the compressed grooves flows along the compressed grooves instead of the core press disposed adjacent to the outer side of the compressed grooves and the diffusion of the body fluid to the outside of the compressed grooves is suppressed.

According to an embodiment, front and rear compressed grooves are formed along the width direction of the absorbent article in front of and behind the area corresponding to the body fluid discharge part of the wearer.

In the embodiment, the front and rear compressed grooves are formed along the width direction of the absorbent article in front of and behind the area corresponding to the body fluid discharge part of the wearer to suppress the diffusion of the body fluid in the longitudinal direction and improve the effect of preventing leakage from the front and rear ends of the absorber. Also, the front and rear compressed grooves function as bending guide lines that guide the absorber to deform along the front and rear round portions of the body.

According to an embodiment, the core press is omitted in positions where the core press overlaps the compressed grooves and in a vicinity of the compressed grooves such that a non-pressed part with a predetermined width is formed around each of the compressed grooves.

In the embodiment, the non-pressed part is formed around each compressed groove so that the body fluid diffused along the core press is temporarily retained in the non-pressed part before reaching the compressed groove. This in turn makes it possible to lower the diffusion rate of the body fluid and enables the body fluid to smoothly diffuse along the compressed groove.

### ADVANTAGEOUS EFFECT OF THE INVENTION

As described above, the present invention makes it possible to suppress the diffusion of a body fluid in the plane direction in a slim absorbent article using a thin absorber and to particularly prevent side leakage of the body fluid.

### BRIEF DESCRIPTION OF THE DRAWINGS

FIG. 1 is a partially-cutaway view of a sanitary napkin 1 according to the present invention;
FIG. 2 is a cross-sectional view taken along line II-II of FIG. 1;
FIG. 3 is a plan view of an absorber 4;
FIG. 4 is a cross-sectional view taken along line IV-IV of FIG. 3;
FIG. 5 is a plan view of an absorber 4 according to a variation;
FIG. 6 is a plan view of an absorber 4 according to a variation;
FIG. 7 is a plan view of an absorber 4 according to a variation;
FIG. 8 is a cross-sectional view of a sanitary napkin 1 according to another embodiment; and
FIG. 9 is a cross-sectional view of a sanitary napkin 1 according to another embodiment.

### DESCRIPTION OF EMBODIMENTS

Embodiments of the present invention are described below with reference to the drawings.

### <BASIC STRUCTURE OF SANITARY NAPKIN>

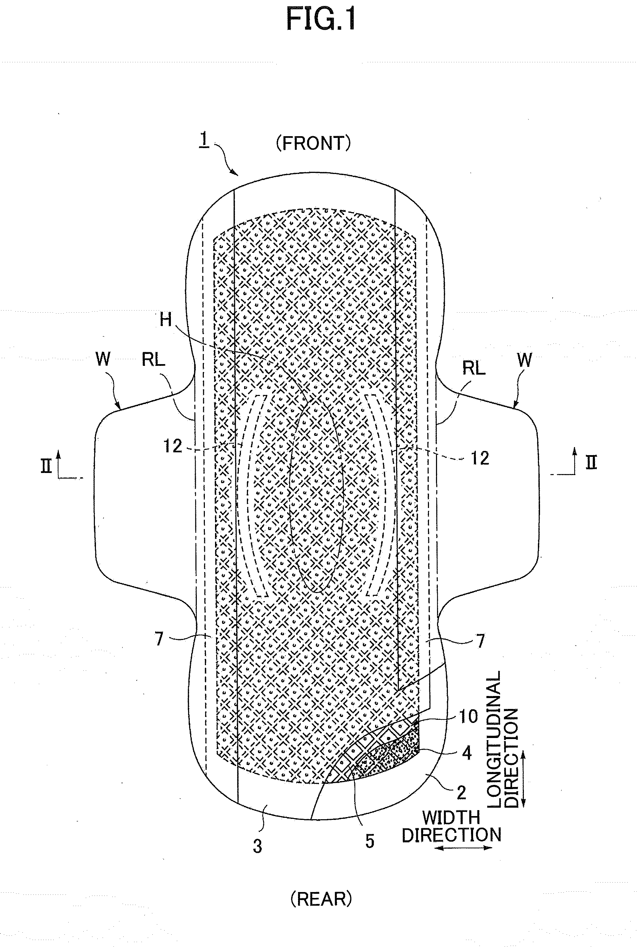

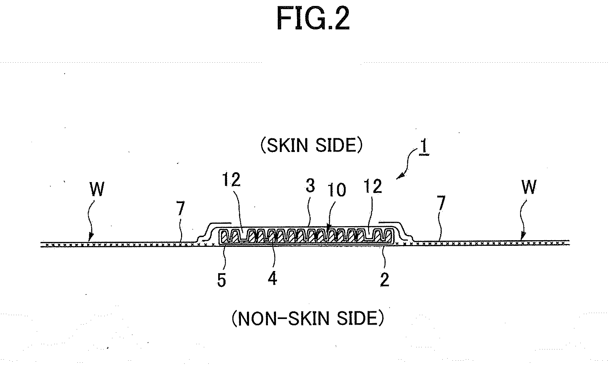

As illustrated in FIGs. 1 and 2, a sanitary napkin 1 according to the present invention includes an impermeable back-side sheet 2 formed of, for example, a polyethylene sheet, a permeable upper-side sheet 3 that allows, for example, menstrual blood and a vaginal discharge (which are hereinafter collectively referred to as a "body fluid") to smoothly pass through, an absorber 4 formed of, for example, cotton pulp or synthetic pulp and disposed between the sheets 2 and 3, and side nonwoven fabrics 7 that are provided on the lateral sides of a skin-contact surface and extend along substantially the entire length in the longitudinal direction. In the periphery of the absorber 4, at the edges in the longitudinal direction of the absorber 4, the outer edges of the back-side sheet 2 and the upper-side sheet 3 are bonded together with an adhesive such as a hot melt or a bonding method such as heat sealing or ultrasonic sealing. Also, at the side edges of the absorber 4, portions of the back-side sheet 2 and the side nonwoven fabrics 7 extending laterally beyond the absorber 4 are bonded together with an adhesive such as a hot melt or a bonding method such as heat sealing or ultrasonic sealing. A pair of right and left wing flaps W are formed at the absorber side positions that correspond to an area H corresponding to a body fluid discharge part in the longitudinal direction. In the illustrated example, to keep the shape of the absorber 4 and improve its diffusivity, the absorber 4 is enveloped in an enveloping sheet 5 formed of, for example, crepe paper or a nonwoven fabric. However, the enveloping sheet 5 may be omitted. Also, although not shown, a second sheet formed of, for example, a hydrophilic nonwoven fabric and having substantially the same shape as the upper-side sheet 3 may be provided adjacent to the non-skin side of the upper-side sheet 3.

Below, the structure of the sanitary napkin 1 is described in more detail. The back-side sheet 2 may be made of a sheet material such as polyethylene having at least water impermeability. The back-side sheet 2 is preferably formed of a material having moisture permeability to prevent stuffiness. As the water-impermeable and moisture-permeable sheet, a microporous sheet is preferably used. A microporous sheet is obtained by melt-mixing an inorganic filler with an olefin resin such as polyethylene or polypropylene to form a sheet, and by stretching the sheet uniaxially or biaxially. On the non-skin side (outer surface) of the back-side sheet 2, one or more streaks of adhesive layers (not shown) are formed along the napkin longitudinal direction to fix the sanitary napkin 1 to underwear when the sanitary napkin 1 is worn on the body. The back-side sheet 2 may also be made of a polyethylene-laminated nonwoven fabric (a nonwoven fabric laminated with a polyethylene film) that is formed by stacking a plastic film and a nonwoven fabric.

The permeable upper-side sheet 3 is preferably made of a porous or nonporous nonwoven fabric or a porous plastic sheet. Examples of fiber materials of the nonwoven fabric include synthetic fibers formed of olefin such as polyethylene or polypropylene, polyester, or polyamide, regenerated fibers such as rayon and cupra, and natural fibers such as cotton. The nonwoven fabric may be produced by any appropriate production method such as spunlacing, spunbonding, thermal bonding, melt-blowing, or needle punching. Among these production methods, spunlacing has an advantage in terms of flexibility and draping characteristics, and thermal bonding has an advantage in terms of bulkiness and compression restoration. When a large number of through holes are formed in the upper-side sheet 3, the sanitary napkin 1 can quickly absorb a body fluid and its dry touch property is improved. Although either long fibers or short fibers may be used for the nonwoven fabric, short fibers are preferably used to give a texture of a towel cloth. Also, to facilitate embossing, olefin fibers made of, for example, polyethylene or polypropylene with a relatively low melting point are preferably used. Also, bicomponent fibers may be used for the nonwoven fabric. Examples of bicomponent fibers include a core-in-sheath fiber including a high-melting-point fiber as a core and a low-melting-point fiber as a sheath, a side-by-side fiber, and a split fiber.

As illustrated in the transverse sectional view of FIG. 2, the width of the upper-side sheet 3 is slightly longer than the width of the absorber 4, and the upper-side sheet 3 merely covers the absorber 4 within a predetermined section that includes the area H corresponding to the body fluid discharge part and extends in the napkin longitudinal direction. The side nonwoven fabrics 7 different from the upper-side sheet 3 are provided outside of the absorber 4. The side nonwoven fabrics 7 are formed of a non-woven fabric material on which a water repellent treatment or a hydrophilic treatment is performed depending on a purpose such as preventing penetration of menstrual blood or a vaginal discharge or improving the feel.

The side nonwoven fabrics 7 may be produced using natural fibers, synthetic fibers, or regenerated fibers as a material according to any appropriate production method. A nonwoven fabric having a reduced basis weight and breathability is preferably used to reduce coarseness and prevent stuffiness. Specifically, a nonwoven fabric produced to have a basis weight of 13-23 g/m² is preferably used. Also, a water-repellent nonwoven fabric coated with a silicone-based, paraffin-based, or alkylchromic chloride-based water repellent is preferably used to effectively prevent permeation of a body fluid.

As illustrated in FIG. 2, an outer portion of each of the side nonwoven fabrics 7 located outside of a middle portion in the width direction is bonded with an adhesive such as a hot melt across a section from a predetermined inner position to an outer edge of the back-side sheet 2 to form a predetermined flap at a predetermined position. The flaps form a pair of right and left wing flaps W on the sides positions that correspond to the area H corresponding to the body fluid discharge part in the longitudinal direction. Adhesive layers (not shown) are provided on the outer surface of the back-side sheet 2 at positions corresponding to the wing flaps W. When the sanitary napkin 1 is attached to shorts, the wing flaps W are folded back toward the opposite side along folding lines RL (FIG. 1) at their base ends and are wound around the crotch of the shorts to fix the sanitary napkin 1. On the other hand, the inside portions of the side nonwoven fabrics 7 are stacked on the skin side of the upper-side sheet 3 and bonded to the absorber 4 (the skin side of the upper-side sheet 3).

### <ABSORBER>

The absorber 4 disposed between the back-side sheet 2 and the upper-side sheet 3 is formed of, for example, pulp and a superabsorbent polymer. The superabsorbent polymer is mixed in the pulp constituting the absorber in the form of, for example, granular powder. The pulp may be made of cellulose fibers such as chemical pulp or dissolving pulp made from wood, or synthetic cellulose fibers such as rayon or acetate. In terms of the function and the price, softwood pulp with a long fiber length is more preferable than hardwood pulp.

The sanitary napkin 1 is a thin slim napkin using the absorber 4 with a low basis weight. The basis weight of the pulp is 50-300 g/m², and is preferably 80-220 g/m². The basis weight of the superabsorbent polymer is 30-180 g/m², and is preferably 50-160 g/m². The basis weights of the pulp and the superabsorbent polymer are not necessarily constant, and may be changed depending on the portions of the absorber. For example, as described later in detail, a high absorption part 15 where the basis weights of the pulp and the superabsorbent polymer are high may be formed in an area corresponding to the area H that corresponds to the body fluid discharge part of the wearer.

Synthetic fibers may also be added to the absorber 4. The synthetic fibers may be made of, for example, polyolefin such as polyethylene or polypropylene, polyester such as polyethylene terephthalate or polybutylene terephthalate, polyamide such as nylon, or a copolymer of these polymers. Also, a mixture of two types of synthetic fibers may be used. Also, bicomponent fibers may be used. Examples of bicomponent fibers include a core-in-sheath fiber including a high-melting-point fiber as a core and a low-melting-point fiber as a sheath, a side-by-side fiber, and a split fiber. When using hydrophobic fibers as the synthetic fibers, the hydrophobic fibers are preferably surface-treated with a hydrophilic agent to have an affinity to the body fluid.

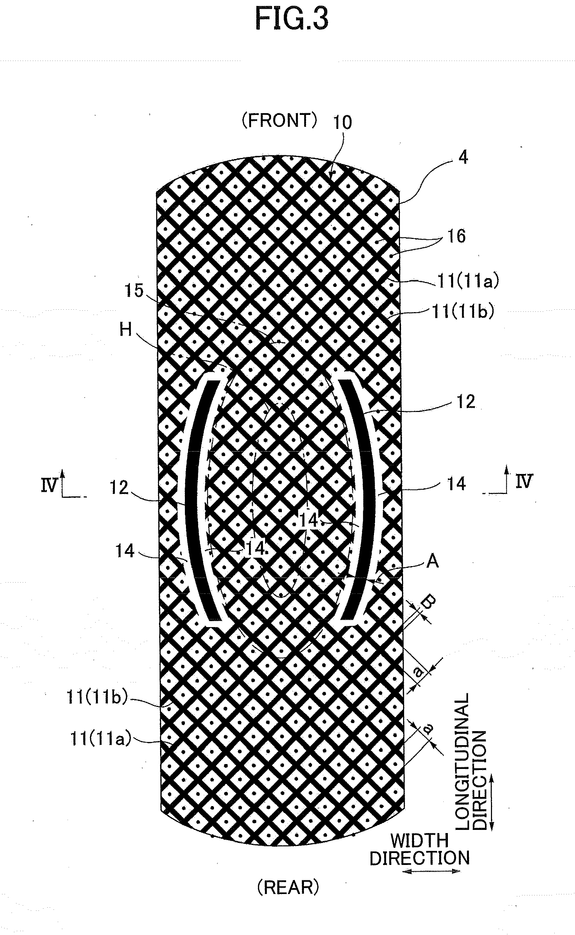

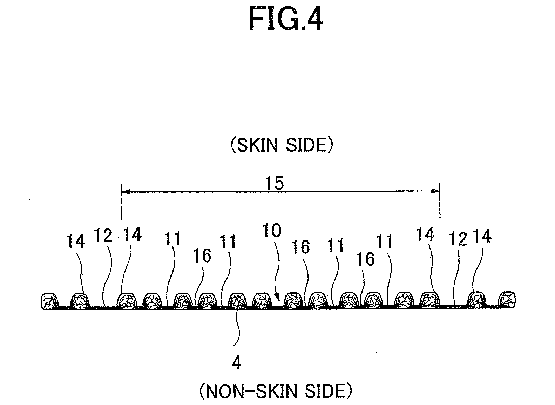

As illustrated in FIGs. 3 and 4, a core press 10 depressed toward the back-side sheet 2 (non-skin side) is formed in at least a surface (skin side surface) of the absorber 4 facing the upper-side sheet 3. The core press 10 is formed to reduce the thickness of the absorber 4 in the thickness direction. The core press 10 is formed by compressing the absorber 4 in a predetermined pattern at predetermined intervals instead of compressing the entire surface of the absorber 4. Because core-press-formed portions of the absorber 4 where the core press 10 is formed are compressed in the thickness direction, fibers in portions where the core press 10 is not formed are drawn by the core-press-formed portions, and the portions where the core press 10 is not formed are influenced and compressed to some extent. As a result, the thickness of the entire absorber 4 is reduced in the thickness direction. The "thickness of the absorber 4" indicates the thickness of the entire absorber 4, and indicates the maximum thickness measured at the thickest portion of the absorber 4.

When the thickness of the absorber 4 before forming the core press 10 is t₀ and the thickness of the absorber 4 after forming the core press 10 is t, the ratio t/t₀ of the thickness after being reduced by forming the core press 10 is preferably about 1/10 to about 7/10. If the ratio is less than 1/10, the absorber becomes hard and the wearing comfort is reduced. On the other hand, if the ratio is greater than 7/10, the effect of compression by the core press 10 becomes small and the thickness of the absorber 4 increases. The ratio may be changed by adjusting, for example, the compression depth of the core press 10, the interval between adjacent core presses, and the plane pattern of the core press 10.

In the illustrated example, the core press 10 is formed by compressing the absorber 4 only from the skin side (the surface facing the upper-side sheet 3). As a result, U-shaped recesses are formed on the skin side of the absorber 4, and the non-skin side (the surface facing the back-side sheet 2) of the absorber 4 becomes substantially flat. Such processing may be performed by passing the absorber through a gap between a patterned roller having multiple protrusions on its surface and an anvil roller having a flat surface. On the other hand, although not shown, U-shaped recesses may be formed on both of the skin side and the non-skin side of the absorber 4 by compressing the absorber 4 from both of the skin side and the non-skin side. Such processing may be performed by passing the absorber 4 through a gap between a first patterned roller having multiple protrusions on its surface and a second patterned roller having protrusions formed at positions corresponding to the protrusions of the first patterned roller. When at least the skin side of the absorber 4 is compressed, a body fluid entering through the upper-side sheet 3 flows into the recesses of the core press 10, can smoothly diffuse along the core press 10, and can be absorbed by the absorber 4 while diffusing.

As illustrated in FIG. 3, the core press 10 is preferably formed in the same pattern on substantially the entire absorber 4. That is, the core press 10 is also formed in the area H corresponding to the body fluid discharge part of the wearer in the same pattern as in the other areas. This makes it possible to reduce the thickness of the entire absorber 4, to effectively reduce the wearing discomfort, and to effectively reduce the influence on the outer appearance.

The core press 10 can be formed in any pattern as long as the thickness of the absorber 4 can be reduced in the thickness direction. However, as illustrated in FIG. 3, the core press 10 preferably includes multiple linear pressed parts 11 that are continuous or intermittent in a predetermined portion and formed between edges of the absorber 4. The linear pressed parts 11 are not formed in a middle portion that does not reach the outer edges of the absorber 4. Instead, each linear pressed part 11 is formed between one of the front, rear, right, and left edges of the absorber 4 and another one of the front, rear, right, and left edges. Forming the linear pressed parts 11 between the edges of the absorber 4 makes it possible to compress the entire absorber 4 and to effectively reduce its thickness.

The core press 10 preferably includes a grid-like pattern composed of first linear pressed parts 11a that are inclined to one side in the width direction and arranged in the longitudinal direction at predetermined intervals and second linear pressed parts 11b that are inclined to the other side in the width direction to intersect the first linear pressed parts 11a and arranged in the longitudinal direction at predetermined intervals. Forming the core press 10 in a grid-like pattern makes it possible to more effectively reduce the thickness of the absorber 4, and to more effectively reduce the wearing discomfort and suppress the influence on the outer appearance. The first linear pressed parts 11a and the second linear pressed parts 11b, respectively, are preferably arranged at substantially the same interval such that a large number of rectangular cells having a square or rhombic shape and defined by the linear pressed parts 11a and 11b are arranged. Although the substantially rectangular cells surrounded by the linear pressed parts 11a and 11b are not directly compressed, their thickness is influenced by the linear pressed parts 11a and 11b and is made smaller than the original thickness.

The intersecting angle between the first linear pressed parts 11a and the second linear pressed parts 11b is substantially a right angle in the illustrated example. However, the intersecting angle may be between about 30 degrees and about 150 degrees. The first linear pressed parts 11a and the second linear pressed parts 11b are preferably inclined with respect to the width direction of the sanitary napkin 1. With this configuration, when the right and left legs are moved back and forth during, for example, walking and a force is applied to the sanitary napkin 1 in a torsional direction due to a pressure from the inside of the groin, the absorber 4 can smoothly deform in an oblique direction along the linear pressed parts 11a and 11b and can smoothly follow the movement of the body. Also, because a body fluid flows in the width direction of the sanitary napkin 1 along the linear pressed parts 11a and 11b, this configuration can prevent side leakage compared with a case where linear pressed parts are formed to extend in a direction parallel to the width direction. To facilitate deformation in the oblique direction and prevent side leakage as described above, the linear pressed parts 11a and 11b are preferably inclined at an angle greater than or equal to 30 degrees with respect to the width direction of the sanitary napkin 1.

To effectively reduce the thickness of the absorber 4, as illustrated in the FIG. 3, a length "a" of each side of the cells defined by the first linear pressed parts 11a and the second linear pressed parts 11b is preferably between 4 mm and 10 mm. If the length "a" is less than 4 mm, the distance between the linear pressed parts 11a and 11b becomes too close. As a result, the absorber 4 becomes hard, and the wearing comfort is reduced. On the other hand, if the length "a" is greater than 10 mm, the central portion of each cell bulges toward the skin side, and the thickness of the entire absorber 4 cannot be sufficiently reduced. The width of each of the linear pressed parts 11a and 11b is preferably between 0.5 mm and 3 mm.

To effectively reduce the thickness of the absorber 4, as illustrated in FIGs. 3 and 4, a central pressed part 16 shaped like, for example, a circular or polygonal dot may be formed in the central portion of each cell which is apart from the linear pressed parts 11a and 11b. This configuration makes it possible to suppress the central portion of each cell from bulging toward the skin side, and makes it possible to effectively reduce the thickness of the entire absorber 4 without much increasing the hardness of the absorber 4. Also, a body fluid penetrated into each cell can smoothly diffuse toward the central pressed part 16 with high density due to capillary action. This in turn makes it possible to improve the diffusivity and to quickly absorb the body fluid. When the central pressed part 16 is formed, the length "a" of each side of the cell may be made greater than the length in a case where the central pressed part 16 is not formed. For example, even if the length "a" is increased up to about 15 mm, the thickness of the absorber 4 can be reduced without problem.

The planar shape of each cell defined by the core press 10 may be a polygon having five or more sides instead of a quadrangle such as a square or a rhombus described above. In the above embodiment, the linear pressed parts 11a and 11b are formed as continuous lines. However, the linear pressed parts 11a and 11b may be formed as discontinuous lines composed of multiple dots arranged at intervals.

As illustrated in FIGs. 3 and 4, on the surface of the absorber 4 facing the upper-side sheet 3, in addition to the core press 10, right and left compressed grooves 12 are formed along the longitudinal direction of the sanitary napkin 1 on at least the sides of the area H corresponding to the body fluid discharge part of the wearer. Forming the compressed grooves 12 makes it possible to block the body fluid diffusing in the width direction of the sanitary napkin 1 from the area H corresponding to the body fluid discharge part of the wearer through the core press 10, and to change the direction of the flow to a direction along the longitudinal direction of the sanitary napkin 1 in which the compressed grooves 12 extend. This in turn makes is possible to prevent the body fluid from diffusing to the lateral edges of the absorber 4, and to reliably prevent the side leakage.

Similarly to the core press 10, the compressed grooves 12 may be composed of U-shaped recesses that are formed only on the skin side of the absorber 4 by compressing the absorber 4 only from the skin side, or composed of U-shaped recesses formed on both of the skin side and the non-skin side of the absorber 4 by compressing the absorber 4 from the skin side and the non-skin side.

In the embodiment illustrated in FIG. 3, the compressed grooves 12 are formed on the sides of the area H corresponding to the body fluid discharge part of the wearer to extend in the longitudinal direction. That is, the compressed grooves 12 are composed of arc lines that are disposed apart from each other in the width direction on the right and left sides of the central portion of the sanitary napkin 1. The compressed grooves 12 extend in the longitudinal direction on the sides of the area H corresponding to the body fluid discharge part of the wearer and have a length that is substantially the same as the area H corresponding to the body fluid discharge part. Each of the compressed grooves 12 preferably has a curved shape that protrudes to the outermost position in the width direction at the center in the longitudinal direction.

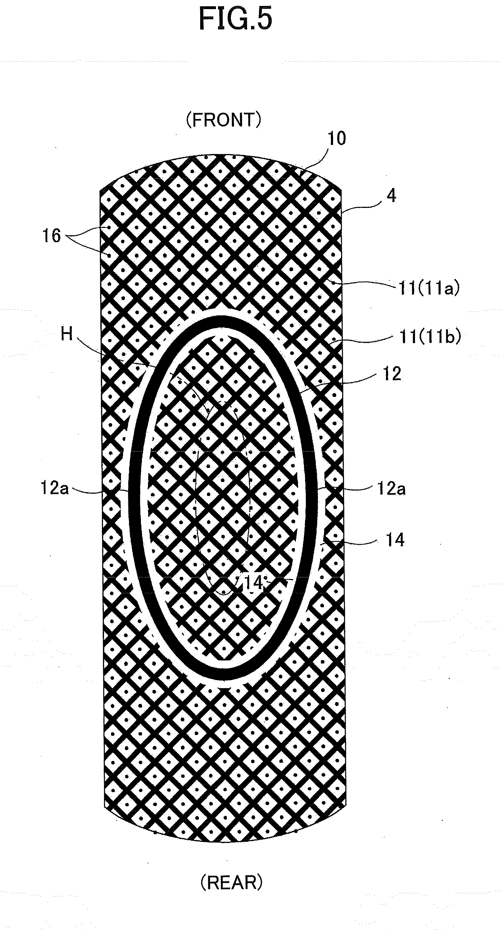

As a variation, as illustrated in FIG. 5, the compressed groove 12 may be formed as an annular line that surrounds the area H corresponding to the body fluid discharge part of the wearer. This configuration makes it possible to suppress the diffusion in the longitudinal direction in addition to the diffusion in the width direction, and therefore makes it possible to effectively prevent the leakage from the lateral and longitudinal edges of the absorber 4. In this case, the compressed groove 12 preferably has a circular, elliptical, or oblong planar shape.

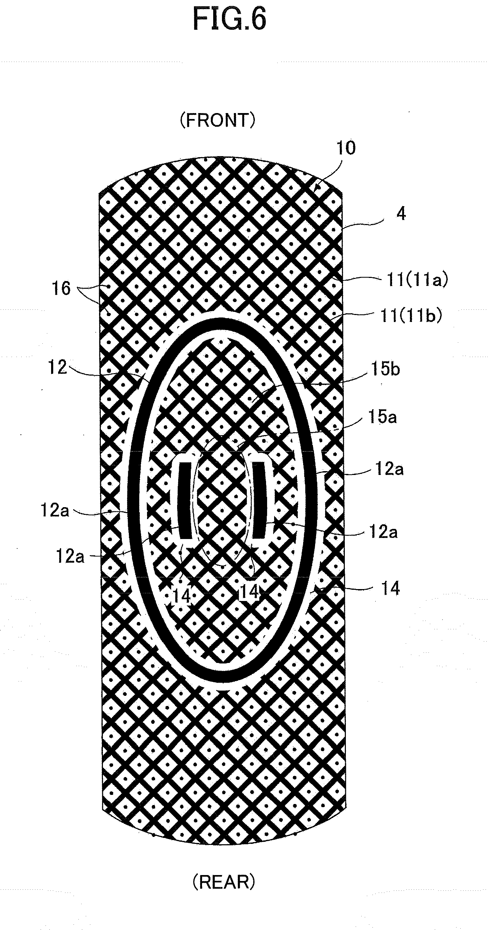

As illustrated in the FIG. 6, the compressed groove 12 preferably includes multiple longitudinal compressed grooves 12a on each side of the area H corresponding to the body fluid discharge part of the wearer. The longitudinal compressed grooves 12a extend in the longitudinal direction and are apart from each other in the width direction. In the example illustrated in FIG. 6, two longitudinal compressed grooves 12a, which are apart from each other in the width direction, are formed on each side of the area H corresponding to the body fluid discharge part of the wearer. One of the two longitudinal compressed grooves 12a located on the outer side is implemented by a longitudinally-extending portion of the annular line surrounding the area H corresponding to the body fluid discharge part of the wearer, and another one of the two longitudinal compressed grooves 12a located on the inner side is implemented by one of arc lines that are disposed apart from each other in the width direction on the right and left sides of the central portion of the sanitary napkin 1. This configuration makes it possible to more reliably prevent the diffusion of the body fluid in the width direction. The multiple longitudinal compressed grooves 12a are preferably arranged concentrically around substantially the center of the area H corresponding to the body fluid discharge part of the wearer, and preferably have substantially parallel curved shapes that protrude outward. To prevent the diffusion of the body fluid in the width direction, the compressed grooves 12a in the innermost positions in the width direction are preferably positioned on the sides of an area that substantially corresponds to the vaginal opening of the wearer.

In this case, the longitudinal compressed groove 12a disposed in the laterally-outer position preferably extends in the forward and backward longitudinal directions longer than the longitudinal compressed groove 12a disposed in the laterally-inner position. With this configuration, the body fluid diffusing outward in the width direction around an end of the longitudinal compressed groove 12a disposed in the laterally-inner position can be blocked by the longitudinal compressed groove 12a disposed in the laterally-outer position, and the diffusion of the body fluid in the laterally-outward direction can be prevented more reliably.

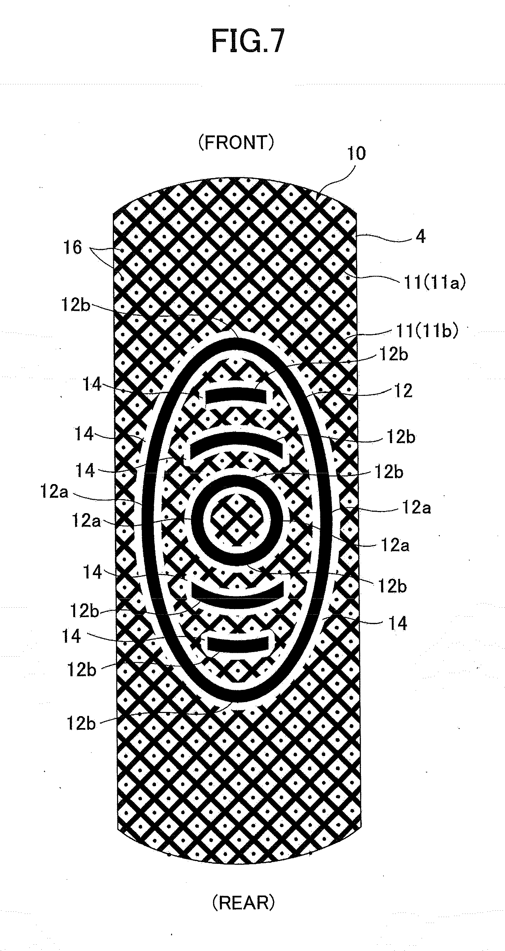

Also, as illustrated in FIG. 7, the longitudinal compressed grooves 12a disposed in the laterally-inner positions may be implemented by longitudinally-extending side portions of a circular annular line disposed to surround an area corresponding to the vaginal opening of the wearer. That is, the compressed grooves may be formed in a pattern composed of double annular lines where a compressed groove 12 formed of a circular annular line surrounding an area corresponding to the vaginal opening is disposed inside of a compressed groove 12 formed of an elliptical annular line that is long in the longitudinal direction and surrounds the area H corresponding to the body fluid discharge part.

As illustrated in FIG. 7, one or more front lateral compressed grooves 12b and one or more rear lateral compressed grooves 12b may be formed in front of and behind the area H corresponding to the body fluid discharge part of the wearer. The front and rear lateral compressed grooves 12b extend in the width direction of the sanitary napkin 1 and are spaced in the longitudinal direction. Providing the front and rear lateral compressed grooves 12b extending in the width direction makes it possible to prevent the diffusion of the body fluid in the longitudinal direction and to prevent the leakage from the front and rear ends of the absorber 4. Also, when the sanitary napkin 1 is worn, the front and rear lateral compressed grooves 12b function as bending guide lines that guide the absorber 4 to deform along the front and rear round portions of the body. The front and rear lateral compressed grooves 12b may be implemented by portions of the annular line extending in the width direction, or may be implemented by independent arc lines extending in the width direction and disposed apart from each other in the longitudinal direction of the sanitary napkin 1. Also, as in the illustrated example, multiple lateral compressed grooves 12b, "four" in this example, may be formed by combining an annular line and arc lines. When four lateral compressed grooves 12b are formed on each of the front and rear sides as in the illustrated example, the outermost lateral compressed groove 12b is preferably implemented by a portion of an elliptical annular line extending in the width direction, the innermost lateral compressed groove 12b is preferably implemented by a portion of a circular annular line extending in the width direction, and two lateral compressed grooves 12b in the middle are preferably implemented by independent arc lines. With this configuration, the innermost annular compressed groove can suppress the diffusion of the body fluid in the vicinity of the vaginal opening; when the body fluid sufficiently diffuses to saturate the area and diffuses outside of the area, the middle arc-shaped lateral compressed grooves 12b can suppress the outward diffusion of the body fluid in the longitudinal direction; and the body fluid can be reliably retained in an area surrounded by the outermost annular compressed groove.

As illustrated in FIGs. 3 and 5-7, the core press 10 is preferably omitted in positions where the core press 10 overlaps the compressed grooves 12 and in the vicinity of the compressed grooves 12 such that non-pressed parts 14 with a predetermined width are formed around the compressed grooves 12. That is, the core press 10 is not formed in areas corresponding to the compressed grooves 12 and in areas with a predetermined with around the compressed grooves 12 so that non-pressed areas (non-pressed parts 14) are formed between the compressed grooves 12 and the core press 10. With the non-pressed parts 14 formed around the compressed grooves 12, the body fluid diffused along the core press 10 is temporarily retained in the non-pressed parts 14 before reaching the compressed grooves 12. This configuration makes it possible to quickly lower the diffusion rate of the body fluid diffusing along the core press 10, and enables the body fluid to smoothly change its direction and flow along the compressed grooves 12. Also, because even the outer sides of the compressed grooves 12 are not connected to the core press 10, the body fluid diffusing along the compressed grooves 12 is prevented from diffusing outward along the core press 10, and the body fluid can be reliably kept within the area surrounded by the compressed grooves 12.

The non-pressed part 14 is formed around the entire periphery of each compressed groove 12 so that the compressed groove 12 and the core press 10 are not directly connected to each other. The width of the non-pressed part 14 is preferably between 0.5 mm and 5 mm to ensure its function as a body fluid buffer zone and prevent the thickness of the absorber 4 from increasing.

The non-pressed parts 14 are preferably formed also around the longitudinal compressed grooves 12a and the lateral compressed grooves 12b. Hereinafter, the term "compressed groove(s) 12" may also encompass the longitudinal compressed grooves 12a and the lateral compressed grooves 12b.

As illustrated in FIG. 3, a groove width A of the compressed groove 12 is preferably greater than a groove width B of the linear pressed part 11 of the core press 10 (A> B). This configuration makes the body fluid diffusing along the compressed groove 12 less likely to move to the core press 10, and makes it possible to suppress the body fluid from diffusing outside of the compressed groove 12. The ratio A/B between the groove widths A and B is preferably between 1.5 and 3.

In forming the core press 10 and the compressed grooves 12 on the absorber 4, the core press 10 and the compressed grooves 12 may be simultaneously formed using a patterned roll on which protrusions corresponding to the core press 10 and the compressed grooves 12 are formed. Alternatively, the compressed grooves 12 may be formed after forming the core press 10.

In the sanitary napkin 1, as illustrated in FIGs. 3 and 4, the high absorption part 15, which has an absorption capacity per unit area greater than that of other parts, is formed at least between the right and left compressed grooves 12 of the absorber 4. The area between the right and left compressed grooves 12 includes the area H corresponding to the body fluid discharge part of the wearer. As illustrated in the FIG. 3, when the compressed grooves 12 implemented by independent arc lines are disposed on the right and left sides, the high absorption part 15 is formed in an area that includes the area sandwiched between the compressed grooves 12. Also, as illustrated in FIG. 5, when the compressed groove 12 is implemented by an annular line formed to surround the area H corresponding to the body fluid discharge part, the high absorption part 15 is formed in an area surrounded by the compressed groove 12. Further, as illustrated in FIGs. 6 and 7, when compressed grooves are formed inside of a compressed groove implemented by an annular line formed to surround the area H corresponding to the body fluid discharge part of the wearer, the high absorption part 15 is formed within an area surrounded by the outermost compressed groove 12. Forming the high absorption part 15 at least between the right and left compressed grooves 12 indicates that when the independent arc-shaped compressed grooves 12 are formed on the right and left sides as illustrated in FIG. 3, the high absorption part 15 may be formed to extend to one or both of areas before and after the area sandwiched between the compressed grooves 12. In the illustrated example, the high absorption part 15 is formed to extend to both of the areas. The high absorption part 15 is formed in at least a part of the area between the right and left compressed grooves 12, and is preferably formed in the entire area. As illustrated in FIGs. 5 through 7, when the compressed groove 12 is implemented by an annular line surrounding the area H corresponding to the body fluid discharge part of the wearer, the high absorption part 15 is preferably formed in substantially the entire area surrounded by the compressed groove 12.

The high absorption part 15 has a greater absorption capacity per unit area than other parts. Providing the high absorption part 15 in the area H corresponding to the body fluid discharge part of the wearer makes it possible to sufficiently absorb and retain a discharged body fluid by the high absorption part 15, and makes it possible to suppress the body fluid from diffusing out of the high absorption part 15. This configuration also makes it possible to prevent the area surrounded by the compressed groove 12 from becoming out of capacity (saturated) and prevent the body fluid from flowing and diffusing out of the area.

The absorption capacity per unit area is measured as described below.
(1) While the napkin is in a taut state (a state where the napkin is spread along a plane and has no wrinkle or fold), the napkin is cut into the high absorption part 15 and other parts, and the areas of these parts are measured. Before this step, the back-side sheet 2 and the upper-side sheet 3 are removed.
(2) The parts are placed in separate mesh bags, and the mouths of the mesh bags are closed. Then, the mesh bags are immersed in a 0.9 wt% physiological saline solution and left for 30 minutes.
(3) The parts are dehydrated with a centrifugal dehydrator (10G) for 3 minutes.
(4) The absorption capacity per unit area of each of the parts is calculated according to the following formula. absorption capacity per unit area (g/cm²) = ((weight after dehydration (g) - (weight before immersion in liquid (g)) / area of part (cm²))

The absorption capacity per unit area of the high absorption part 15 measured as described above is preferably between 0.3 g/cm² and 2.0 g/cm², and preferably between 0.3 g/cm² and 1.0 g/cm². Also, the absorption capacity per unit area of the high absorption part 15 is 1.5 to 5 times, preferably 1.5 to 2.5 times, greater than the absorption capacity per unit area of other parts.

The absorption capacity per unit area may be increased, for example, by increasing the basis weight of the pulp and the superabsorbent polymer or by changing the type of the superabsorbent polymer to one having a greater absorption amount. In the case where the basis weight of the pulp and the superabsorbent polymer is increased, if only the basis weight of the superabsorbent polymer is increased, the corresponding area becomes hard, and the wearer is likely to feel the graininess of the superabsorbent polymer. Therefore, it is preferable to also increase the basis weight of the pulp by substantially the same ratio as the increased ratio of the superabsorbent polymer. The increased basis weight of the pulp and the superabsorbent polymer in the high absorption part 15 is 1.5 to 5 times, preferably 1.5 to 2.5 times, greater than that of other areas. As a result of increasing the basis weight of the pulp and the superabsorbent polymer, the thickness of the high absorption part 15 increases compared to other areas. However, because the core press 10 is also formed in the high absorption part 15, even if the thickness slightly increases, the wearing comfort is not influenced. Specifically, although it varies depending on the thickness of the entire absorber 4, the difference between the thickness of the high absorption part 15 and the thickness of other areas is preferably less than or equal to 1 mm.

When the absorption capacity per unit area is increased by increasing the basis weight of the pulp and the superabsorbent polymer, the rigidity of the high absorption part 15 becomes relatively high and the rigidity of other parts becomes relatively low. This achieves an effect that while the area H corresponding to the body fluid discharge part of the wearer is in close contact with the body, other parts can smoothly follow the movement of the body.

When the absorption capacity per unit area is increased by changing the types of superabsorbent polymers, a superabsorbent polymer with a large water absorption amount measured according to JIS K7223 may be used for the high absorption part 15.

When multiple longitudinal compressed grooves 12a are formed at least on each side of the area H corresponding to the body fluid discharge part of the wearer as illustrated in FIG. 6, the absorption capacity per unit area of the absorber in the inner area inside of the longitudinal compressed grooves 12a may be made higher than that in the outer area outside of the longitudinal compressed grooves. By gradually lowering the absorption capacity per unit area from the inner area toward the outer area bordered by the longitudinal compressed grooves 12a, the body fluid is sufficiently absorbed in the inner area before diffusing to the outer area. Thus, this configuration makes it possible to further suppress the diffusion of the body fluid and to more reliably prevent the side leakage. In the example illustrated in FIG. 6, two longitudinal compressed grooves 12a are formed on each side. A central portion between the inner longitudinal compressed grooves 12a is set as a high absorption part 15a with the highest absorption capacity per unit area, an annular portion between the outer longitudinal compressed grooves 12a and the inner longitudinal compressed grooves 12a is set as a middle absorption part 15b that has an absorption capacity per unit area lower than that of the high absorption part 15a in the center, and a portion outside of the outer longitudinal compressed grooves 12a (the compressed groove 12) is set as a low absorption part with the lowest absorption capacity per unit area.

In the sanitary napkin 1 configured as described above, the thickness of the absorber 4 is reduced in the thickness direction by forming the core press 10 on the absorber 4. Thus, the sanitary napkin 1 is made as a slim napkin that does not much affect the outer appearance. However, depending on the use condition, the body fluid may diffuse along the core press 10 and may particularly leak from the lateral edges. For this reason, the left and right compressed grooves 12 are formed along the longitudinal direction of the sanitary napkin 1 on at least the sides of the area H corresponding to the body fluid discharge part of the wearer to block the body fluid diffusing toward the lateral edges of the absorber 4. Also, the high absorption part 15 with an absorption capacity per unit area higher than that of other parts is formed at least between the right and left compressed grooves 12 of the absorber 4 so that a discharged body fluid can be prevented from diffusing in the plane direction and can be absorbed and retained in the absorber 4.

### <OTHER EMBODIMENTS>

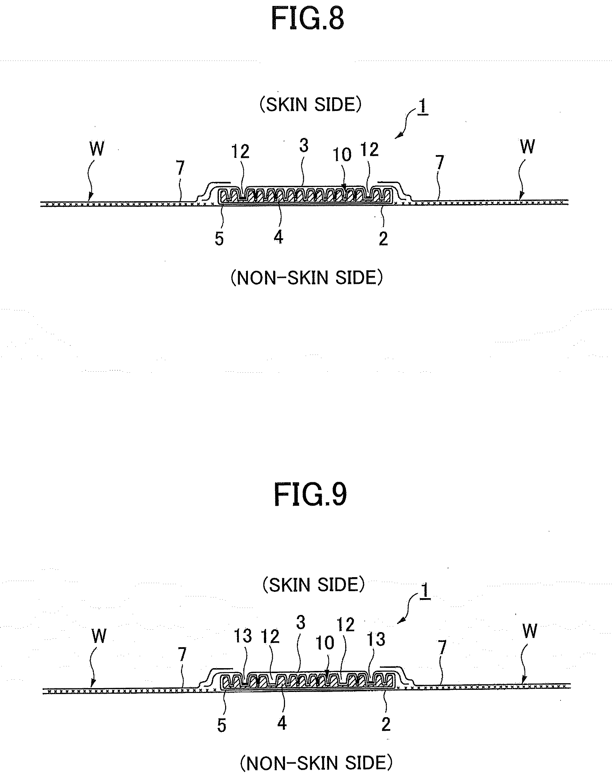

In the above embodiment, as illustrated in FIG. 2, the compressed grooves 12 are formed by compressing only the absorber 4 or both of the enveloping sheet 5 and the absorber 4 from the skin side of the absorber 4, and the skin side of the compressed grooves 12 is covered by the upper-side sheet 3 such that spaces are formed in the compressed grooves 12. Alternatively, as illustrated in FIG. 8, the compressed grooves 12 may be formed by compressing the upper-side sheet 3 and the absorber 4 together from the skin side of the upper-side sheet 3 in a state where the upper-side sheet 3 is placed on the skin side of the absorber 4 so that the upper-side sheet 3 is present on the side surface and the bottom of each compressed groove 12. With the configuration where the compressed groove 12 is formed by compressing the upper-side sheet 3 and the absorber 4 together, the body fluid flowing outward in the width direction on the surface of the upper-side sheet 3 enters the recess of the compressed groove 12 and is absorbed by the absorber 4 through the compressed groove 12. This configuration makes it possible to prevent side leakage caused by the lateral flow of the body fluid on the surface. Also, by compressing the upper-side sheet 3 and the absorber 4 together, the upper-side sheet 3 and the absorber 4 between the right and left compressed grooves 12 are united and closely contact the body fluid discharge part of the wearer. This in turn makes it possible to improve the fit on the body, improve the body fluid absorption capability, and improve the wearing comfort.

Also, as illustrated in FIG. 9, after forming the right and left compressed grooves 12 by compressing only the absorber 4 or both of the enveloping sheet 5 and the absorber 4 from the skin side of the absorber 4 and stacking the upper-side sheet 3 on the skin side of the absorber 4, compressed grooves 13 may be formed by compressing the upper-side sheet 3 and the absorber 4 together from the skin side of the upper-side sheet 3 such that the upper-side sheet 3 is present on the side surface and the bottom of each compressed groove 13. The compressed grooves 13 are disposed outside of and apart from the compressed grooves 12 in the width direction. Providing the compressed grooves 13 formed by compressing the upper-side sheet 3 and the absorber 4 together at positions outside of the compressed grooves 12 formed by compressing only the absorber 4 makes it possible to further improve the leakage prevention effect.

The present application is based on and claims priority to Japanese Patent Application No. 2018-038352 filed on March 5, 2018.

### EXPLANATION OF REFERENCE NUMERALS

- 1: sanitary napkin
- 2: back-side sheet
- 3: upper-side sheet
- 4: absorber
- 5: enveloping sheet
- 7: side nonwoven fabric
- 10: core press
- 11: linear pressed part
- 11a: first linear pressed part
- 11b: second linear pressed part
- 12: compressed groove
- 12a: longitudinal compressed groove
- 12b: lateral compressed groove
- 13: compressed groove
- 14: non-pressed part
- 15: high absorption part
- 16: central pressed part

## Claims

1. An absorbent article (1) being a sanitary napkin, comprising:
an absorber (4) disposed between a permeable upper-side sheet (3) and an impermeable back-side sheet (2), wherein
a core press (10) depressed toward the back-side sheet (2) is formed on at least a surface of the absorber (4) facing the upper-side sheet (3) in a pattern at predetermined intervals such that a thickness of the absorber (4) is reduced in a thickness direction;
right and left compressed grooves (12) having a curved shape are formed along a longitudinal direction of the absorbent article (1) on at least sides of an area corresponding to a body fluid discharge part of a wearer, wherein the compressed grooves (12) are formed from an annular line or arc lines; and
a high absorption part is formed at least in an inner area inside of the right and left compressed grooves (12) of the absorber (4), in an inner area an absorption capacity per unit area of the high absorption part being greater than an absorption capacity per unit area of the absorber (4) in an outer area outside of the right and left compressed grooves (12),
wherein
multiple longitudinal compressed grooves (12a) extending in the longitudinal direction and disposed apart from each other in a width direction are formed on at least each side of the area corresponding to the body fluid discharge part of the wearer, and
the high absorption part is formed by making a basis weight of pulp and a superabsorbent polymer included in the inner area of the absorber (4) greater than a basis weight of the pulp and the superabsorbent polymer included in the outer area of the absorber (4) or by using a superabsorbent polymer having a greater absorption amount than an absorption amount of a superabsorbent polymer used in the outer area.

2. The absorbent article (1) as claimed in claim 1, wherein the compressed grooves (12) are formed to surround the area corresponding to the body fluid discharge part of the wearer.

3. The absorbent article (1) as claimed in claim 1, wherein the core press (10) includes multiple linear pressed parts (11a, 11b) that are formed between edges of the absorber (4) and are continuous or omitted in the compressed grooves (12) .

4. The absorbent article (1) as claimed in claim 3, the core press (10) includes
multiple first linear pressed parts (11a) that are arranged at predetermined intervals in the longitudinal direction and inclined to one side in a width direction, and
multiple second linear pressed parts (11b) that are arranged in the longitudinal direction at predetermined intervals, inclined to another side in the width direction, and intersect the first linear pressed parts (11a).

5. The absorbent article (1) as claimed in claim 3, wherein a groove width of the compressed grooves (12) is greater than a groove width of the linear pressed parts.

6. The absorbent article (1) as claimed in claim 1, wherein front and rear compressed grooves (12b) are formed along a width direction of the absorbent article (1) in front of and behind the area corresponding to the body fluid discharge part of the wearer.

7. The absorbent article (1) as claimed in claim 1, wherein the core press (10) is omitted in positions where the core press (10) overlaps the compressed grooves and in a vicinity of the compressed grooves (12) such that a non-pressed part (14) with a predetermined width is formed around each of the compressed grooves (12).

## Patentansprüche

1. Ein absorbierender Artikel (1), der eine Damenbinde ist, umfassend:
einen Absorber (4), der zwischen einer durchlässigen Oberseitenlage (3) und einer undurchlässigen Rückseitenlage (2) angeordnet ist, wobei
eine Kernpresse (10), die in Richtung der Rückseitenlage (2) eingedrückt ist, auf mindestens einer Oberfläche des Absorbers (4), die der Oberseitenlage (3) zugewandt ist, in einem Muster in vorbestimmten Intervallen ausgebildet ist, so dass eine Dicke des Absorbers (4) in einer Dickenrichtung reduziert ist;
rechte und linke komprimierte Rillen (12) mit einer gekrümmten Form entlang einer Längsrichtung des absorbierenden Artikels (1) an mindestens Seiten eines Bereichs ausgebildet sind, der einem Körperflüssigkeitsabgabebereich eines Trägers entspricht, wobei die komprimierten Rillen (12) aus einer ringförmigen Linie oder Bogenlinien ausgebildet sind; und
ein Teil mit hoher Absorption zumindest in einem inneren Bereich innerhalb der rechten und linken komprimierten Rillen (12) des Absorbers (4) ausgebildet ist, wobei in einem inneren Bereich eine Absorptionskapazität pro Flächeneinheit des Teils mit hoher Absorption größer ist als eine Absorptionskapazität pro Flächeneinheit des Absorbers (4) in einem äußeren Bereich außerhalb der rechten und linken komprimierten Rillen (12),
wobei
mehrere komprimierte Längsrillen (12a), die sich in der Längsrichtung erstrecken und in einer Breitenrichtung voneinander beabstandet sind, auf mindestens jeder Seite des Bereichs ausgebildet sind, der dem Körperflüssigkeitsabfuhrteil des Trägers entspricht, und
der Teil mit hoher Absorption gebildet wird, indem ein Flächengewicht von Zellstoff und einem superabsorbierenden Polymer, das in dem inneren Bereich des Absorbers (4) enthalten ist, größer gemacht wird als ein Flächengewicht des Zellstoffs und des superabsorbierenden Polymers, das in dem äußeren Bereich des Absorbers (4) enthalten ist, oder indem ein superabsorbierendes Polymer verwendet wird, das eine größere Absorptionsmenge hat als eine Absorptionsmenge eines superabsorbierenden Polymers, das in dem äußeren Bereich verwendet wird.

2. Absorbierender Artikel (1) nach Anspruch 1, wobei die komprimierten Rillen (12) so geformt sind, dass sie den Bereich umgeben, der dem Körperflüssigkeitsabgabebereich des Trägers entspricht.

3. Absorbierender Artikel (1) nach Anspruch 1, wobei die Kernpresse (10) mehrere lineare Pressteile (11a, 11b) umfasst, die zwischen den Rändern des Absorbers (4) ausgebildet sind und in den komprimierten Rillen (12) durchgehend sind oder weggelassen werden.

4. Saugfähiger Artikel (1) nach Anspruch 3, wobei die Kernpresse (10) umfasst
mehrere erste lineare Pressteile (11a), die in vorbestimmten Abständen in Längsrichtung angeordnet und in Breitenrichtung zu einer Seite geneigt sind, und
mehrere zweite lineare Pressteile (11b), die in Längsrichtung in vorbestimmten Abständen angeordnet und in Breitenrichtung zu einer anderen Seite hin geneigt sind und die ersten linearen Pressteile (11a) kreuzen.

5. Saugfähiger Artikel (1) nach Anspruch 3, wobei eine Rillenbreite der verdichteten Rillen (12) größer ist als eine Rillenbreite der linearen Pressteile.

6. Absorptionsartikel (1) nach Anspruch 1, wobei vordere und hintere komprimierte Rillen (12b) entlang einer Breitenrichtung des Absorptionsartikels (1) vor und hinter dem Bereich ausgebildet sind, der dem Körperflüssigkeitsabgabebereich des Trägers entspricht.

7. Absorbierender Artikel (1) nach Anspruch 1, wobei die Kernpresse (10) an Positionen, an denen die Kernpresse (10) die komprimierten Rillen überlappt, und in der Nähe der komprimierten Rillen (12) weggelassen wird, so dass ein nichtgepresster Teil (14) mit einer vorbestimmten Breite um jede der komprimierten Rillen (12) herum gebildet wird.

## Revendications

1. Article absorbant (1) étant une serviette hygiénique, comprenant :
un absorbeur (4) disposé entre une feuille perméable côté supérieur (3) et une feuille imperméable côté verso (2), sachant que
un renfoncement d'âme (10) enfoncé vers la feuille côté verso (2) est formé sur au moins une surface de l'absorbeur (4) faisant face à la feuille côté supérieur (3) dans un motif à intervalles prédéterminés de telle sorte qu'une épaisseur de l'absorbeur (4) soit réduite dans une direction d'épaisseur ;
des rainures comprimées (12) droite et gauche ayant une forme courbe sont formées le long d'une direction longitudinale de l'article absorbant (1) sur au moins des côtés d'une zone correspondant à une partie d'évacuation de fluide corporel d'une porteuse, sachant que les rainures comprimées (12) sont formées à partir d'une ligne annulaire ou de lignes d'arc ; et
une partie à haute absorption est formée au moins dans une zone intérieure à l'intérieur des rainures comprimées (12) droite et gauche de l'absorbeur (4), dans une zone intérieure une capacité d'absorption par unité de surface de la partie à haute absorption étant supérieure à une capacité d'absorption par unité de surface de l'absorbeur (4) dans une zone extérieure à l'extérieur des rainures comprimées (12) droite et gauche,
sachant que
de multiples rainures comprimées (12a) longitudinales s'étendant dans la direction longitudinale et disposées de manière espacée les unes par rapport aux autres dans une direction de largeur sont formées sur au moins chaque côté de la zone correspondant à la partie d'évacuation de fluide corporel de la porteuse, et
la partie à haute absorption est formée en faisant en sorte qu'un poids de base de pulpe et d'un polymère superabsorbant inclus dans la zone intérieure de l'absorbeur (4) soit supérieur à un poids de base de la pulpe et du polymère superabsorbant inclus dans la zone extérieure de l'absorbeur (4) ou en utilisant un polymère superabsorbant ayant une quantité d'absorption supérieure à une quantité d'absorption d'un polymère superabsorbant utilisé dans la zone extérieure.

2. L'article absorbant (1) tel que revendiqué dans la revendication 1, sachant que les rainures comprimées (12) sont formées pour entourer la zone correspondant à la partie d'évacuation de fluide corporel de la porteuse.

3. L'article absorbant (1) tel que revendiqué dans la revendication 1, sachant que le renfoncement d'âme (10) inclut de multiples parties pressées linéaires (11a, 11b) qui sont formées entre des bords de l'absorbeur (4) et sont continues ou omises dans les rainures comprimées (12).

4. L'article absorbant (1) tel que revendiqué dans la revendication 3, sachant que le renfoncement d'âme (10) inclut
de multiples premières parties pressées linéaires (11a) qui sont agencées à intervalles prédéterminés dans la direction longitudinale et inclinées vers un côté dans une direction de largeur, et
de multiples deuxièmes parties pressées linéaires (11b) qui sont agencées dans la direction longitudinale à intervalles prédéterminés, inclinées vers un autre côté dans la direction de largeur, et croisent les premières parties pressées linéaires (11a).

5. L'article absorbant (1) tel que revendiqué dans la revendication 3, sachant qu'une largeur de rainure des rainures comprimées (12) est supérieure à une largeur de rainure des parties pressées linéaires.

6. L'article absorbant (1) tel que revendiqué dans la revendication 1, sachant que des rainures comprimées (12b) avant et arrière sont formées le long d'une direction de largeur de l'article absorbant (1) devant ou derrière la zone correspondant à la partie d'évacuation de fluide corporel de la porteuse.

7. L'article absorbant (1) tel que revendiqué dans la revendication 1, sachant que le renfoncement d'âme (10) est omis dans des positions où le renfoncement d'âme (10) chevauche les rainures comprimées et à une proximité des rainures comprimées (12) de telle sorte qu'une partie non pressée (14) ayant une largeur prédéterminée soit formée autour de chacune des rainures comprimées (12).
